**Europäisches Patentamt**

**European Patent Office**

**Office européen des brevets**

(19)

(11) Veröffentlichungsnummer: **0 131 606**
**B1**

(12) # EUROPÄISCHE PATENTSCHRIFT

(45) Veröffentlichungstag der Patentschrift:
05.11.86

(21) Anmeldenummer: 84900503.8

(22) Anmeldetag: 07.01.84

(86) Internationale Anmeldenummer:
PCT/EP 84/00004

(87) Internationale Veröffentlichungsnummer:
WO 84/02720 (19.07.84 Gazette 84/17)

(51) Int. Cl.⁴: **C 12 Q 1/42, G 01 N 33/566**

(54) **VERFAHREN UND REAGENZ ZUR DIFFERENZIERTEN BESTIMMUNG VON ISOENZYMEN DER ALKALISCHEN PHOSPHATASE.**

(30) Priorität: 12.01.83 GB 8300685
23.05.83 GB 8314165

(43) Veröffentlichungstag der Anmeldung:
23.01.85 Patentblatt 85/4

(45) Bekanntmachung des Hinweises auf die Patenterteilung:
05.11.86 Patentblatt 86/45

(84) Benannte Vertragsstaaten:
AT BE CH DE FR GB LI LU NL SE

(56) Entgegenhaltungen:
EP-A-0 032 204
US-A-4 030 995
US-A-4 166 766

Chemical Abstracts, vol. 93, no. 25, 22 December 1980, Columbus, Ohio, (US) F.G. Lehmann: "Differentiation of human alkaline phosphatases by lectin binding affinity", see page 333, column 1, abstract 233620n
Chemical Abstracts, vol. 94, no. 1, 5 January 1981, Columbus, Ohio, (US), F.G. Lehmann: "Human alkaline phosphatases, Evidence of three isoenzymes (placental, intestinal and liver-bone-kidney, type) by lectin binding affinity and immunological specificity", see page 151, column 2, abstract 1460c
Chemical Abstracts, vol. 95, no. 15, 12 October 1981,

(73) Patentinhaber: Boehringer Mannheim GmbH, Sandhofer Strasse 116, D-6800 Mannheim 31 (DE)

(72) Erfinder: ROSALKI, Sidney, Bertram, 78 Loudoun Road St. Johns Wood, London NW8 (GB)

(74) Vertreter: Daum, Martin, Dr., Boehringer Mannheim GmbH Patentabteilung Postfach 31 01 20, D-6800 Mannheim 31 (DE)

(56) Entgegenhaltungen: (Fortsetzung)
Columbus, Ohio, (US) D.T. Dorai et al.: "Fractionation of sialoglycoproteins on an immobilised sialic acid-binding lectin", see page 286, column 1, abstract 128562p
Clinical Chemistry, vol.28, no.10, October 1982, Washington (US), D.W.Moss:" Alkaline Phosphatase Isoenzymes", see pages 2007-2016.

## Beschreibung

Die alkalische Phosphatase (Ortho phosphoric monoester phosphohydrolase) katalysiert die Hydrolyse von Phosphorsäure-monoestern bei alkalischen pH-Werten. Dieses Enzym kommt in vielen Geweben vor. Es existieren gewebespezifische Formen (Isoenzyme) beispielsweise in der Leber, in den Knochen, in dem Dünndarm, in den Nieren und in der Placenta. Von diesen Organen werden die Isoenzyme an das Blutplasma abgegeben. Die charakteristischen Eigenschaften der jeweiligen Isoenzyme bleiben beim Übergang vom erzeugenden Organ in das Blutplasma erhalten.

Das Blutplasma gesunder Menschen enthält hauptsächlich das Leber- und Knochen-Isoenzym. Bei ungefähr einem Viertel aller Personen wird im Plasma auch alkalische Phosphatase gefunden, die im Dünndarm entstanden ist. Der Anteil dieses Dünndarm-Isoenzyms liegt in diesen Fällen bei einem Mittelwert von 10 % des gesamten alkalischen Phosphatase-Gehaltes im Plasma. Während der Schwangerschaft, insbesondere während der letzten 3 Monate wird auch in der Placenta entstehende alkalische Phosphatase an das Blutplasma abgegeben. Die gesamte alkalische Phosphatase-Aktivität des Plasmas ergibt sich als Summe der Aktivitäten aller gewebespezifischen Einzelkomponenten.

Die Bestimmung der Gesamt-Aktivität der alkalischen Phosphatase sowie insbesondere der Aktivität des Leber- und Knochen-Isoenzyms im Plasma ist von besonderer diagnostischer Bedeutung bei der Untersuchung von Krankheiten der Leber und des Knochensystems. Die Erkrankungen bewirken einen Anstieg der alkalischen Phosphataseaktivität in Plasma, da unter diesen Voraussetzungen von der Leber bzw. den Knochen in verstärktem Maße alkalische Phosphatase an das Plasma abgegeben wird. Der Anstieg der alkalischen Phosphatase-Aktivität des Leber- oder des Knochen-Isoenzyms im Plasma kann demnach dazu dienen, eine Erkrankung dieser Gewebe zu erkennen. Bei einer Erkrankung der Leber entsteht zusätzlich eine weitere Enzymvariante der alkalischen Phosphatase, die durch Komplexbildung des Leber-Isoenzyms mit Lipiden oder Proteinen entsteht und die vermutlich im Gallen-Trakt gebildet wird. Dieses sogenannte Gallen-Isoenzym, das auch als hochmolekulare, schnelle oder $\alpha$-1alkalische Phosphatase bezeichnet wird, kann in diesen Fällen im Plasma als eine alkalische Phosphatase-Komponente mit geringerem Anteil erscheinen.

Die Differenzierung der alkalischen Phosphatase-Isoenzyme aus Dünndarm, Placenta, Nieren und Galle voneinander oder auch von dem Leber- und dem Knochen-Isoenzym ist ohne weiteres möglich, da sich diese gewebespezifischen Formen in ihren chemischen, physikalischen und immunologischen Eigenschaften deutlich unterscheiden. Verfahren zur Abtrennung und Bestimmung dieser Isoenzyme sind bekannt und beruhen beispielsweise auf dem unterschiedlichen Verhalten der Isoenzyme gegen Inhibitoren, auf Unterschiede in der elektrischen Ladung usw. So können beispielsweise die Unterschiede in der elektrophoretischen Beweglichkeit in alkalischen Puffersystemen zur Trennung dieser Isoenzyme herangezogen werden.

Nur geringe Unterschiede sind jedoch zwischem dem Leber- und dem Knochenzym zu beobachten. Insbesondere unterscheiden sich das Leber- und das Knochen-Isoenzym der alkalischen Phosphatase nur wenig hinsichtlich der elektrischen Ladung, der Hitzestabilität und in der Antwort auf Inhibitoren (beispielsweise gegenüber Harnstoff). Diese Unterschiede genügen nicht, um eine brauchbare Differenzierung zwischen diesen beiden Enzymformen und eine hinreichende Quantifizierung jeder Form in einem Gemisch beider Formen zu erreichen.

In F. G. Lehmann, Klin. Wochenschr. 58, 947-951 (1980) wird die Trennung gereinigter, menschlicher, alkalischer Phosphatase mittels Affinitätschromatographie an Weizenkeimlectin, Linsenlectin und Concanavalin A beschrieben. Es gelingt auf diese Weise, zwischen drei Isoenzymklassen zu differenzieren:

dem Placenta

dem Dünndarm- und

dem Leber-Knochen-Nieren-Isoenzymtyp.

Der Leber-Knochen-Nieren-Isoenzymtyp zeigt eine gewisse Mikroheterogenität. Eine Differenzierung innerhalb dieser Isoenzymklasse gelingt jedoch nicht.

Nach F. G. Lehmann, Biochim. Biophys. Acta 616, 41-59 (1980) zeigen Antikörper gegen alkalische Phosphatase aus Leber, Knochen oder Nieren 100 %ige Kreuzreaktion. Eine Unterscheidung gelang folglich auch auf diesem Wege nicht.

Aufgabe der Erfindung war es, ein neues Verfahren bereitzustellen, mit dessen Hilfe eine Unterscheidung und differenzierte Bestimmung des Leber- und Knochen-Isoenzyms der alkalischen Phosphatase ermöglicht wird. Gelöst wird diese Aufgabe durch das erfindungsgemäße Verfahren, bei dem die Probe, beispielsweise Blutplasma oder Serum, mit einem Lektin, das N-Acetylglucosamin-Reste zu binden vermag, versetzt und inkubiert wird. Danach wird eine Trennung der Lektingebundenen von den freien Isoenzym-Anteilen durchgeführt und die alkalische Phosphatase-Aktivität in einem oder in beiden getrennten Medien bestimmt.

Lektine sind Kohlenhydrat-bindende Proteine. Als Lektine eignen sich für das erfindungsgemäße Verfahren besonders Lektine von Weizenkeimen (Triticum vulgaris) oder von Kartoffeln (Solanum tuberosum). Besonders bevorzugt ist das Weizenkeim-Lektin, das als lyophilisiertes Pulver im Handel erhältlich ist (Sigma London Chemical Co.). Lektin wird vorzugsweise in Form eines Lösung, vorzugsweise in Wasser, der Probe zugesetzt. Die Konzentration solcher Lektinlösungen beträgt vorzugsweise 0,1 - 1 % (Gew./Vol.). Solche Lektine binden das Knochen-Isoenzym schneller als das Leber-Isoenzym. Durch die Bindung an das Lektin werden die physikalischen, chemischen und auch immunologischen Eigenschaften der Isoenzyme verändert, so daß eine Trennung der Lektingebundenen von den freien Isoenzym-Amteilen mit Hilfe hierzu geeigneter, üblicher Methoden möglich wird.

Nach dem Zusatz der Lektin-Lösung zu der Serum- bzw. Plasmaprobe wird die Mischung inkubiert. Die Inkubationszeit beträgt üblicherweise 10 bis 60, vorzugsweise 20 bis 40 Minuten. Die Temperatur wird während der Inkubation konstant gehalten, und zwar in einem Bereich, bei dem die Enzymaktivität nicht beeinflußt wird. Bevorzugt wird die Inkubation bei 20 bis 45° C durchgeführt.

Danach wird die an Lektin gebundene alkalische Phosphatase-Aktivität von der nicht gebundenen getrennt. Die Trennung kann beispielsweise mit Hilfe der Elektrophorese erfolgen. Hierzu wird eine Plasma- oder Serumprobe mit einer äquivalenten Menge einer Lektin-Lösung versetzt, einige Zeit stehengelassen und danach einer elektrophoretischen Trennung unterworfen. Das Knochenenzym wandert wegen seiner Bindung an das Lektin bei der Elekrophorese nicht, während das Leberenzym in seinem elektrophoretischen Verhalten nicht beeinträchtigt wird. Während demnach in nicht mit Lektin behandelten Proben die beiden Isoenzyme nicht feststellbare Unterschiede in ihrer Wanderungsgeschwindigkeit aufweisen, so kann nach der Lektinbehandlung aufgrund der Wanderungsgeschwindigkeitsunterschiede klar zwischen den beiden Isoenzymformen unterschieden werden.

Die getrennten Zonen können in bekannter Weise angefärbt und bestimmt werden, beispielsweise durch densitometrische Messung der Elektrophorese-Membran.

Die elektrophoretische Trennung wird vorzugsweise mit Hilfe von Celluloseacetat-Membranen durchgeführt. Es können aber auch Membranen aus anderen Materialien, beispielsweise aus Agarose, Agargel oder aus einem Polyacrylamidgel eingesetzt werden.

Die elektrophoretische Trennung zwischen dem Knochen- und Leber-Isoenzym kann noch verbessert werden, wenn das Lektin in einer Pufferlösung enthalten ist. Als Puffer hat sich besonders ein Tris-barbital-Puffer als geeignet erwiesen. Die Konzentration des Lektins in der Pufferlösung beträgt vorzugsweise 1 bis 10 % (Gew./Vol.). Nach dieser Ausführungsform des erfindungsgemäßen Verfahrens wird die Elektrophoresemembran zunächst mit der Lektinhaltigen Pufferlösung getränkt und dann mit der Probelösung versetzt und inkubiert. Die Elektrophorese wird mit derselben Pufferlösung, jedoch ohne Lektinzusatz, durchgeführt. Es wird eine klare Trennung der Knochen- und Leber-Isoenzym-Fraktion erhalten.

Da die Zugabe von Lektin auch bewirkt, daß das an Lektin gebundene Isoenzym ausfällt, kann auch eine Trennung durch Zentrifugation erreicht werden. Enthält die Probe nahezu ausschließlich Knochen-Isoenzym, so zeigt der Zentrifugationsüberstand eine vernachlässigbare alkalische Phosphatase-Aktivität. Enthält die Serumprobe hauptsächlich Leber-Isoenzym, so wird durch die Zugabe von Lektin nur ein relativ geringer Anteil der alkalischen Phosphatase-Aktivität ausgefällt. Mit Hilfe von Seren mit bekanntem Gehalt an Knochen- und Leber-Isoenzymen können Bezugswerte ermittelt werden, anhand derer die Aktivität an Knochen- bzw. Leber-Isoenzym in unbekannten Proben ermittelt werden kann.

Mit Hilfe der Elektrophorese konnte gezeigt werden, daß die alkalische Phosphatase-Aktivität im Überstand hauptsächlich von dem Leber-Isoenzym herrührt. In Kenntnis der Tatsache, daß die gesamte Menge an Knochen-Isoenzym und nur ein kleiner und relativ konstanter Anteil an Leber-Isoenzym durch die Zugabe von Lektin und anschließender Zentrifugation aus einer Mischung der beiden Isoenzyme entfernt werden, kann die Menge an Leber-Isoenzym der alkalischen Phosphatase in einer Mischung des Knochen- und Leber-Isoenzyms in unbekannten Verhältnissen leicht bestimmt werden.

Eine bevorzugte Ausführungsform des erfindungsgemäßen Verfahrens besteht demnach darin, daß die Blut- bzw. Plasmaprobe, gegebenenfalls nach Abtrennen sonstiger Isoenzyme der alkalischen Phosphatase, mit Weizenkeim- oder Kartoffel-Lektin versetzt, inkubiert und danach zentrifugiert wird. Der Überstand wird vom Zentrifugat getrennt. In dem Überstand wird das Leber-Isoenzym bestimmt, wobei zu berücksichtigen ist, daß ein kleiner, jedoch konstanter Anteil, des Leber-Isoenzyms jeweils mitausgefällt wird. Die Berücksichtigung erfolgt zweckmäßigerweise mit Hilfe von Bezugswerten, die anhand von Proben mit bekanntem Gehalt an Leber-Isoenzymen erhalten worden sind.

Zur Bestimmung des Knochen-Isoenzyms wird das Zentrifugat resuspendiert, zweckmäßigerweise mit einer Salzlösung, beispielsweise einer Lösung von Natriumchlorid in Wasser. Die Salzkonzentration beträgt vorzugsweise 0,5 - 1 % (Gew./Vol.). Den Niederschlag wieder zu resuspendieren hat den weiteren Vorteil, daß in dieser Suspension das Dünndarm-Isoenzym nicht mehr enthalten ist. Vorteilhafterweise wird soviel Salzlösung zugesetzt, daß das ursprüngliche Volumen der Probe wieder erreicht wird. In der so erhaltenen Suspension wird die alkalische Phosphatase-Aktivität in bekannter Weise bestimmt. Mit Hilfe von Vergleichsproben kann gezeigt werden, daß sich ungefähr 80 % des Knochen-Isoenzyms in dieser Suspension befinden. Durch Vergleich mit Bezugswerten, die anhand von Proben mit bekanntem Isoenzym-Gehalt ermittelt worden sind, konnte gezeigt werden, daß sich durch Multiplikation der in der Suspension gefundenen Aktivität mit dem Faktor 1.25 eine gute Korrelation der Werte, die nach dem erfindungsgemäßen Verfahren für das Knochen-Isoenzym gefunden werden, mit Werten, die nach anderen Methoden erhalten werden, ergibt.

Die Aktivität des Leber-Isoenzyms erhält man mit guter Genauigkeit, indem von der Gesamtaktivität der alkalischen Phosphatase der Wert, der nach vorstehendem Verfahren für das Knochen-Isoenzym gefunden worden ist, abgezogen wird.

Enthält die zu messende Probe Gallen-Isoenzym, so erscheint dies teilweise im Niederschlag. Es ist in diesen Fällen zweckmäßig, das Gallen-Isoenzym vor der Durchführung des erfindungsgemäßen Verfahrens zu entfernen. Dies kann erreicht werden durch Zugabe der Lektine Conc valin A oder von Rizin. Diese Lektine binden selektiv das Gallen-Isoenzym, ohne einen Bindungseffekt auf das Leber- oder Knochen-Isoenzym zu zeigen.

Zweckmäßigerweise wird der Salzlösung, die zur Resuspension des Niederschlags benutzt wird, ein lösungsvermittelnder Stoff, beispielsweise Natriumdodecylsulfat, zugesetzt. Ein solcher Zusatz ist nicht mit einer Inhibierung des Lektin-gebundenen Isoenzyms verbunden. Die Konzentration des lösungsvermittelnden Stoffes in der Salzlösung beträgt 5 - 20 g/l, vorzugsweise 8 - 12 g/l.

Es ist ferner möglich, das freie Isoenzym von dem Lektin-gebundenen Isoenzym-Anteil dadurch zu trennen, daß man eine Lösung von N-Acetylglucosamin zusetzt und die erhaltene Mischung elektrophoretisch trennt. Die Konzenttation von N-Acetylglucosamin beträgt 1 - 50, vorzugsweise 5 - 20 g/l.

Die Trennung zwischen den beiden Isoenzymen kann prinzipiell auch nach anderen bekannten Methoden erfolgen, beispielsweise aufgrund differenzierter Bindungseffekte gegenüber Ionenaustauschern, mit Hilfe der Gelchromatographie oder mit Hilfe der isoelektrischen Fokusierung. Alle diese Trennmethoden beruhen darauf, daß durch die Kombination des Isoenzyms mit dem Lektin das chemische, physikalische und immunologische Verhalten verändert wird, beispielsweise Änderung der elektrischen Ladungsverhältnisse, der Molmasse usw.

Die erfindungsgemäß verwendbaren Lektine, beispielsweise Weizenkeim-Lektin, binden nicht in nennenswertem Maße das Dünndarm-Isoenzym. Sie binden jedoch sehr stark das Gallen-Isoenzym. Das Dünndarm- und Gallen-Isoenzym sind jedoch sehr leicht voneinander und von dem Knochen- und Leber-Isoenzym unterscheidbar und quantifizierbar, beispielsweise durch Elektrophorese, Chromatographie oder aufgrund ihrer Reaktion mit Inhibitoren. Aus der Gesamt-Aktivität und aus det Aktivität des Dünndarm- oder Gallen-Isoenzyms, die getrennt bestimmt werden können, wenn sie anwesend sind, und aus der Kenntnis der unterschiedlichen Effekte eines erfindungsgemäß anwendbaren Lektins auf das Knochen- und Leber-Isoenzym kann die Aktivität jedes einzelnen dieser gewebespezifischen Isoenzyme der alkalischen Phosphatase im Blutplasma oder Serum individuell bestimmt werden.

Ein weiterer Gegenstand der Erfindung ist eine Reagenz zur Durchführung eines Verfahrens zur Differenzierung von Isoenzymen der alkalischen Phosphatase, das dadurch gekennzeichnet ist, daß es ein Lektin enthält, das N-Acetylglucosamin-Reste zu binden vermag. Das Reagenz enthält das Lektin vorwiegend in gelöster Form, vorzugsweise in Form einer wäßrigen Lösung. Die Konzentration an Lektin beträgt vorzugsweise 0,1 - 1 % (Gew./Vol.). Das Reagenz kann jedoch auch in trockener, vorzugsweise lyophilisierter Form vorliegen. Diese Form wird üblicherweise erhalten, indem vorstehend genannte wäßrige Lösungen lyophilisiert werden.

**Beispiel 1**

Eine Probe eines Serums von einem Patienten mit einem Knochen- oder Leberleiden, wird mit der gleichen Menge einer wäßrigen Lösung versetzt, die 5 g Weizenkeim-Lektin in einem Liter Wasser enthält. Als Weizenkeim-Lektin wird lyophilisiertes Pulver aus Triticum vulgaris von der Fa. Sigma London Chemical Co. verwendet. Die Mischung wird 30 Minuten bei 37° C inkubiert. Danach wird eine übliche elektrophoretische Trennung mit Hilfe einer Cellulose-Acetat-Membran durchgeführt. Die alkalische Phosphatase-Aktivität wird in den getrennten Zonen durch übliche Anfärbeverfahren und durch densitometrische Messung bestimmt.

**Beispiel 2**

Gleiche Volumina einer wäßrigen Lektin-Lösung, hergestellt aus 5 g Weizenkeim-Lektin der Fa. Sigma Chemical Co. und einem Liter Wasser, und der zu prüfenden Seren werden vermischt. Die Seren sollen neben dem Knochen- und Leber-Isoenzym nur vernachlässigbare Mengen anderer Isoenzyme der alkalischen Phosphatase enthalten. Sind größere, nicht vernachlässigbare Mengen an anderen Enzymen vorhanden, so sind diese vorher mit Hilfe üblicher bekannter Verfahren abzutrennen. Die Mischungen aus Probe und Lektin-Lösung werden bei 37° C 30 Minuten lang inkubiert und danach zentrifugiert. Der Überstand wird entfernt. Der Niederschlag wird in einer 0,9 %igen (Gew./Vol.) Natriumchlorid-Lösung resuspendiert. Die alkalische Phosphatase-Aktivität in der Suspension wird bestimmt. Die gefundene Aktivität wird mit dem Faktor 1.25 multipliziert und ergibt den Gehalt der Probe an Knochen-Isoenzym an.

In üblicher Weise wird die Gesamtaktivität an alkalischer Phosphatase der Probe bestimmt. Aus der Differenz der Gesamtaktivität und des gemessenen Wertes für das Knochen-Isoenzym ergibt sich der Gehalt an Leber-Isoenzym der Probe.

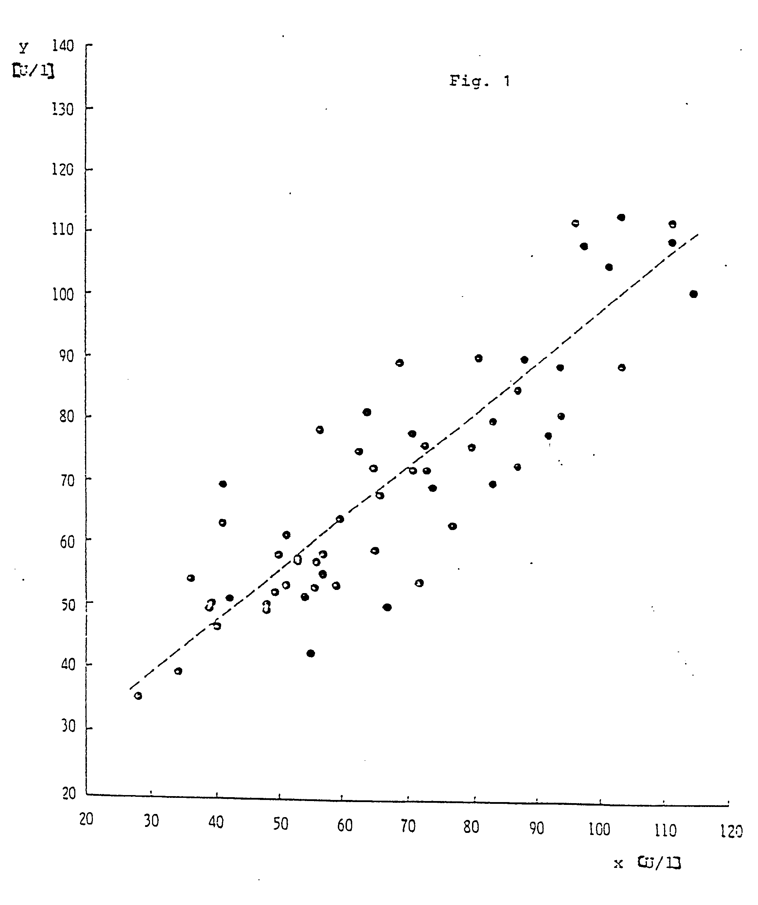

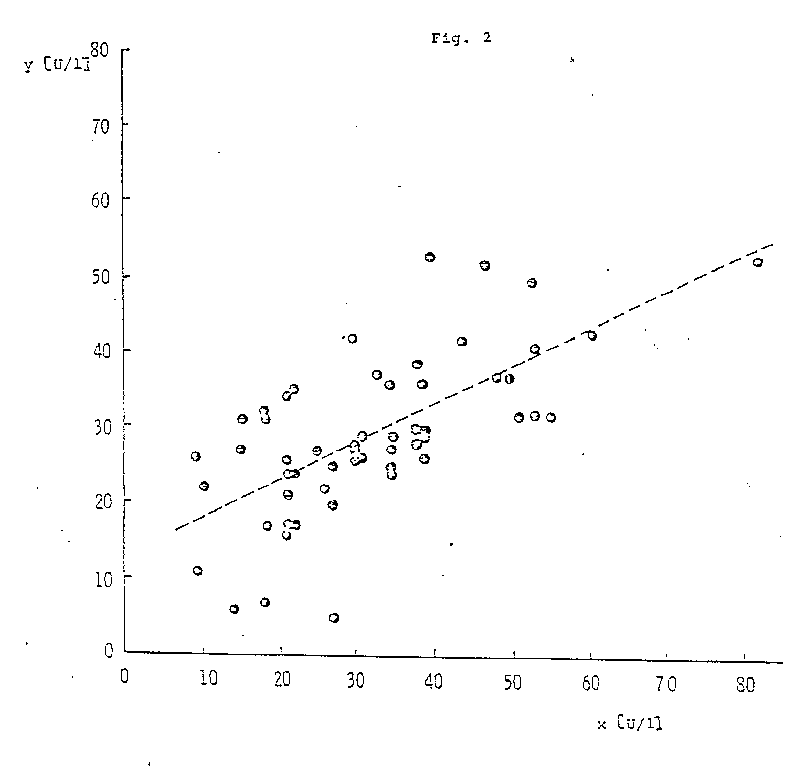

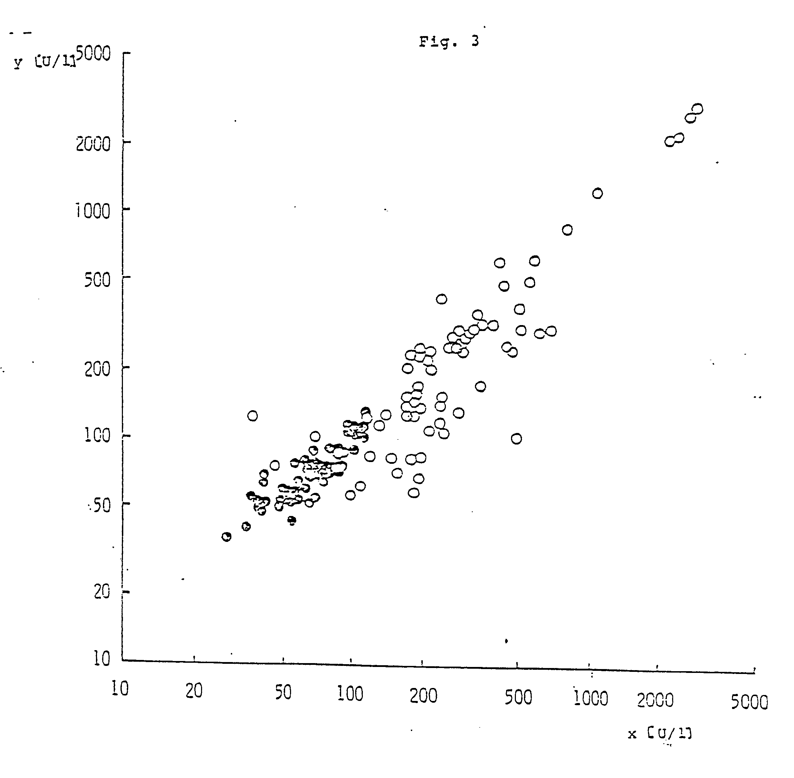

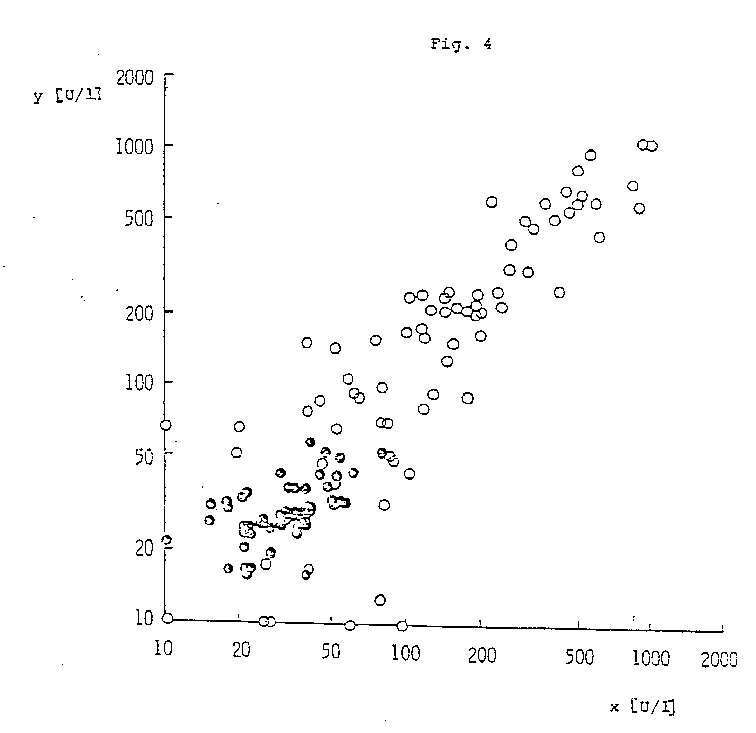

In Fig. 1 bis Fig. 4 sind die nach diesem Verfahren gefundenen Werte für Seren von gesunden und auch von an einem Knochen- oder Leberleiden erkrankten Menschen den Werten gegenübergestellt, die mit Hilfe der konventionellen Methode der aufeinanderfolgenden selektiven Hitze-Inaktivierung ermittelt worden sind.

Im einzelnen stellen dar:

Fig. 1: Vergleich der alkalischen Phosphatase-Aktivität des Knochen-Isoenzyms, bestimmt durch Behandlung mit Weizenkeim-Lektin (y) und durch selektive Hitze Inaktivierung (x). Temperatur 30° C; n = 59 (gesunde Probanden); $y = 0.84 x + 14.03$; $r = 0.89$

Fig. 2: Vergleich der alkalischen Phosphatase-Aktivität des Leber-Isoenzyms, bestimmt durch Behandlung mit Weizenkeim-Lektin (y) und durch selektive Hitze-Inaktivierung (x). Temperatur 30° C; n = 59 (gesunde Probanden); $y = 0.49 x + 13.27$; $r = 0.66$.

Fig. 3: Vergleich der alkalischen Phosphatase-Aktivität für das Knochen-Isoenzym bestimmt durch Behandlung mit Weizenkeim-Lektin (y) und durch selektive Hitze-Inaktivierung (x). Temperatur 50° C; n = 133 gesunde (●) und an einem Knochenleiden erkrankte (O) Probanden; $y = 0.97 x - 9.96$; $r = 0.98$.

Fig. 4: Vergleich der alkalischen Phosphatase-Aktivität des Leber-Isoenzyms, bestimmt durch Behandlung mit Weizenkeim-Lektin (y) umd durch selektive Hitze-Inaktivierung (x). Temperatur 30°C; n = 133 gesunde (●) und an einem Leberleiden erkrankte (O) Probanden; $y = 1.02 x + 14.54$; $r = 0,93$.

**Patentansprüche**

1. Verfahren zur Differenzierung des Knochen- und Leber-Isoenzyms der alkalischen Phosphatase in einer Probe, dadurch gekennzeichnet, daß die Probe mit einem Lektin, das N-Acetylglucosamin-Reste zu binden vermag, versetzt, die erhaltene Mischung inkubiert, danach der Lektingebundene von dem freien Isoenzym-Anteil getrennt und die alkalische Phosphatase-Aktivität in einem oder in beiden der getrennten Medien bestimmt wird.

2. Verfahren gemäß Anspruch 1, dadurch gekennzeichnet, daß als Lektin Weizenkeim- oder Kartoffel-Lektin verwendet wird.

3. Verfahren gemäß Anspruch 1 oder 2, dadurch gekennzeichnet, daß Lektin in Form einer wäßrigen Lösung eingesetzt wird, in der die Lektin-Konzentration 0,1 - 1 % (Gew./Vol.) beträgt.

4. Verfahren gemäß einem der Ansprüche 1 bis 3, dadurch gekennzeichnet, daß die Trennung der Lektin-gebundenen von den freien Isoenzym-Anteilen mit Hilfe der Elektrophorese erfolgt.

5. Verfahren gemäß Anspruch 4, dadurch gekennzeichnet, daß als Elektrophoresemembran eine Membran aus Cellulose-Acetat, Agarose, Agargel oder aus Polyacrylamid-Gel eingesetzt wird.

6. Verfahren gemäß einem der Ansprüche 1 bis 5, dadurch gekennzeichnet, daß die Trennung der Lektin-gebundenen von den freien Isoenzym-Anteilen durch Zentrifugation erfolgt.

7. Verfahren gemäß Anspruch 6, dadurch gekennzeichnet, daß nach der Zentrifugation der Niederschlag wieder in einer Salzlösung resuspendiert wird.

8. Verfahren gemäß Anspruch 7, dadurch gekennzeichnet, daß als Salzlösung eine Natriumchlorid-Lösung einsetzt wird, deren Konzentration 0,5 - 1 % (Gew./Vol.) beträgt.

9. Verfahren gemäß Anspruch 7 oder 8, dadurch gekennzeichnet, daß der Salzlösung ein lösungsvermittelnder Stoff zugesetzt wird.

10. Reagenz zur differenzierten Bestimmung des Knochen- und Leber-Isoenzyms der alkalischen Phosphatase gemäß einem der Ansprüche 1 - 9, dadurch gekennzeichnet, daß es ein Lektin, das N-Acetylglucosamin-Reste zu binden vermag, in Form einer Lösung enthält.

11. Reagenz gemäß Anspruch 10, dadurch gekennzeichnet, daß es als Lektin Weizenkeim- oder Kartoffel-Lektin enthält.

12. Reagenz gemäß Anspruch 10 oder 11, dadurch gekennzeichnet, daß das Lektin in Form einer wäßrigen Lösung eingesetzt wird, in der die Lektin-Konzentration 0,1 - 1 % (Gew./Vol.) beträgt.

**Claims**

1. Process for the differentiation of the bone and liver isoenzymes of alkaline phosphatase in a sample, characterised in that the sample is mixed with a lectin which is able to bind N-acetylglucosamine residues, the mixture obtained is incubated, thereafter the lectin-bound portion is separated from the free isoenzyme portion and the alkaline phosphatase activity is determined in one or in both of the separated media.

2. Process according to claim 1, characterised in that wheat germ or potato lectin is used as lectin.

3. Process according to claim 1 or 2, characterised in that lectin is used in the form of an aqueous solution in which the lectin concentration amounts to o.1 - 1% (wt./vol.).

4. Process according to one of claims 1 to 3, characterised in that the separation of the lectin-bound from the free isoenzyme portions takes place with the help of electrophoresis.

5. Process according to claim 4, characterised in that, as electrophoresis membrane, there is used a membrane of cellulose acetate, agarose, agar gel or of polyacrylamide gel.

6. Process according to one of claims 1 to 5, characterised in that the separation of the lectin-bound from the free isoenzyme portions takes place by centrifuging.

7. Process according to claim 6, characterised in that, after the centrifuging, the precipitate is again resuspended in a salt solution.

8. Process according to claim 7, characterised in that as salt solution there is used a sodium chloride solution, the concentration of which amounts to 0.5-1% (wt./vol.).

9. Process according to claim 7 or 8, characterised in that a solubilising material is added to the salt solution.

10. Reagent for the differentiated determination of the bone and liver isoenzyme of alkaline phosphatase according to one of claims 1 - 9, characterised in that it contains a lectin. which is able to bind N-acetylglucosamine residues, in the form of a solution.

11. Reagent according to claim 10, characterised in that as lectin it contains wheat germ or potato lectin.

12. Reagent according to claim 10 or 11, characterised in that the lectin is used in the form of an aqueous solution in which the lectin concentration amounts to G.1 - 1% (wt./vol.).

agarose, en agargel ou en gel de polyacrylamide.

6. Procédé selon l'une des revendications 1 à 5, caractérisé en ce que la séparation de la fraction d'iso-enzyme liée à la lectine d'avec la fraction d'iso-enzyme libre s'effectue par centrifugation.

7. Procédé selon la revendication 6, caractérisé en ce qu'après la centrifugation le précipité est mis en suspension de nouveau dans une solution saline.

8. Procédé selon la revendication 7, caractérisé en ce que la solution saline utilisée est une solution de chlorure de sodium dont la concentration est de 0,5 à 1 % (poids/volume)

9. Procédé selon l'une des revendications 7 ou 8, caractérisé en ce qu'on ajoute à la solution saline une substance permettant la dissolution.

10. Réactif pour le dosage différentiel de l'iso-enzyme des os et du foie de la phosphatase alcaline selon l'une des revendications 1 à 9, caractérisé en ce qu'il contient, sous forme d'une solution, une lectine capable de fixer les restes de N-acétylglucosamine.

11. Réactif selon la revendication 10, caractérisé en ce que la lectine qu'il contient est de la lectine de germe de blé ou de la lectine de pomme de terre.

12. Réactif selon l'une des revendications 10 ou 11, caractérisé en ce que la lectine est utilisée sous la forme d'une solution aqueuse dans laquelle la concentration de lectine est de 0,1 à 1 % (poids/volume).

**Revendications**

1. Procédé pour la différenciation de l'iso-enzyme des os et du foie de la phosphatase alcaline dans un échantillon, caractérisé en ce que l'échantillon est soumis à l'action d'une lectine capable de fixer les restes de N-acétylglucosamine, que le mélange obtenu est soumis à une incubation, qu'ensuite la fraction d'iso-enzyme liée à la lectine est séparée de la fraction d'iso-enzyme libre et que l'activité de la phosphatase alcaline est déterminée dans l'un des deux milieux séparés ou dans les deux.

2. Procédé selon la revendication 1, caractérisé en ce que la lectine utilisée est de la lectine du germe de blé ou de la lectine de pomme de terre.

3. Procédé selon l'une des revendications 1 ou 2, caractérisé en ce que la lectine est utilisée sous la forme d'une solution aqueuse dans laquelle la concentration de la lectine est de 0,1 à 1 % (poids/volume).

4. Procédé selon l'une des revendications 1 à 3, caractérisé en ce que la séparation de la fraction d'iso-enzyme liée à la lectine d'avec la fraction d'iso-enzyme libre s'effectue au moyen de l'électrophorèse.

5. Procédé selon la revendication 4, caractérisé en ce que la membrane d'électrophorèse utilisée est une membrane en acétate de cellulose, en

Fig. 1

Fig. 2

Fig. 3

Fig. 4